# EUROPEAN PATENT APPLICATION

(11) **EP 4 628 086 A1**
(43) Date of publication of application: **08.10.2025**
(21) Application number: 23879903.5
(22) Date of filing: 04.12.2023
(51) Int. Cl.: A61K 35/32, A61K 31/7105, A61K 48/00, A61P 15/10, A61P 43/00, C12N 5/0775, C12N 15/88, C12N 15/113

(54) **MICROPARTICLE, DRUG FOR PREVENTION OR TREATMENT OF ERECTILE DYSFUNCTION, AND ERECTILE DYSFUNCTION IMPROVEMENT METHOD**

(71) Applicant: Dexon Pharmaceuticals Inc., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2023/043199
(87) International publication number: WO 2024/085261

(57) **Abstract**

Microparticles that contain miRNA that controls the production of nitric oxide and are an agent for promoting the production of nitric oxide are new microparticles that enable controlling the expression of a protein and/or a gene related to erectile dysfunction; a preventive drug or a therapeutic drug for erectile dysfunction; a method for improving erectile dysfunction; it is preferable that the microparticles be exosomes; and it is preferable that the microparticles of the present invention contain miRNA targeting a gene concerning the expression of nitric oxide synthase as the miRNA that controls the production of nitric oxide, and be an agent for promoting the expression of nitric oxide synthase.

## Description

### Technical Field

The present invention relates to microparticles, a preventive drug or a therapeutic drug for erectile dysfunction, and a method for improving erectile dysfunction.

### Background Art

It is widely known that erectile dysfunction (ED) increases with aging. It is estimated that one in three Japanese men aged 40 or over has ED due to various lifestyle diseases and an increase in social stress. Structural pathogenesis accounts for 80% or more of the causes of ED. Among them, the most common cause is vasculogenic disorder, which may participate in arterial inflow disorder and abnormal venous outflow (systemic venous occlusion of the corpora cavernosa).

Phosphodiesterase 5 inhibitor (PDE5I), which is known as an oral drug for ED, has a serious side effect that causes myocardial infarction in rare cases. The development of new therapies has therefore been examined.

For example, Non Patent Literature 1 discloses that human umbilical cord blood stem cells are administered to the penis of a man with severe type 2 diabetes to produce a useful effect on erectile function.

Meanwhile, the investigation of microRNAs (miRNAs) has progressed to reveal the functions and objective genes of miRNAs related to nitric oxide (NO) and nitric oxide synthase (NOS) (see Non Patent Literatures 2 to 5).

Non Patent Literature 2 discloses that miR-16 promotes the activity of inducible nitric oxide synthase (NOS2 or iNOS) to increase the production of NO, required for maintaining the antitumor microenvironment.

Non Patent Literature 3 discloses that miR-155 increases iNOS based on the fact that while the introduction of miR-155 strongly induces iNOS, the suppression of miR-155 reduces iNOS.

Non Patent Literature 4 discloses that the accumulation of Nrf2 in nuclei due to the overexpression of miR-101 increases with HO-1 induction, VEGF expression, and the production of NO derived from endothelial nitric oxide synthase (eNOS).

Non Patent Literature 5 discloses that the overexpression of miR-455-3p in human umbilical vein endothelial cells (HUVECs) increases the amount of protein of eNOS.

### Document List

### Non Patent Literatures

Non Patent Literature 1: Exp Clin Transplant. (2010) 8(2):150-160
Non Patent Literature 2: Int. J. Mol. Sci. (2021) 22(12): 6264
Non Patent Literature 3: Biochem Biophys Res Commun. (2018) 503(2): 452-458
Non Patent Literature 4: Antioxid. Redox Signal. (2014) 21(18): 2469-2482
Non Patent Literature 5: Sci. Rep. (2017) 7: 44807

### Summary of Invention

### Technical Problem

Non Patent Literature 1 has not described miRNA.

Although Non Patent Literatures 2 to 5 describe the functions and objective genes of miRNAs related to nitric oxide (NO) and nitric oxide synthase (NOS), Non Patent Literatures 2 to 5 do not specify means for administering these miRNAs as therapeutic drugs for erectile dysfunction.

An object to be achieved by the present invention is to provide new microparticles that enable controlling (suppressing or inhibiting) the expression of a protein and/or a gene related to erectile dysfunction.

### Solution to Problem

The present inventors have found that microparticles containing specific microRNA enable controlling (suppressing or inhibiting) the expression of a protein and/or a gene related to erectile dysfunction.

The present invention and preferable configurations of the present invention are specifically as follows.

[1] Microparticles, comprising:
miRNA that controls production of nitric oxide,
wherein the microparticles are an agent for promoting production of nitric oxide.

[2] The microparticles according to [1], wherein the microparticles are exosomes.

[3] The microparticles according to [1], comprising:
miRNA targeting a gene concerning expression of nitric oxide synthase as the miRNA that controls the production of nitric oxide,
wherein the microparticles are an agent for promoting the expression of nitric oxide synthase.

[4] The microparticles according to [3], comprising:
at least one selected from the following NOS-related miRNA group as the miRNA targeting the gene concerning the expression of nitric oxide synthase,
wherein the NOS-related miRNA group consists of hsa-miR-101-3p, has-miR-101-5p, has-miR-155-5p, hsa-miR-16-2-3p, has-miR-16-5p, and hsa-miR-455-3p.

[5] The microparticles according to [1], wherein the microparticles are isolated by purification from culture supernatant of dental pulp-derived stem cells.

[6] The microparticles according to [5], wherein the microparticles do not comprise components of the culture supernatant of the dental pulp-derived stem cells other than exosomes.

[7] The microparticles according to [1], comprising the miRNA that controls the production of nitric oxide at higher concentration than the culture supernatant of the dental pulp-derived stem cells.

[8] The microparticles according to [1] for use in administration to a subject with erectile dysfunction and having a score of 10 or less based on the International Index of Erectile Function IIEF-5.

[9] A preventive drug or a therapeutic drug for erectile dysfunction, comprising the microparticles according to any one of [1] to [8].

[10-0] A method for improving erectile dysfunction, comprising: administering an effective amount of the microparticles according to any one of [1] to [8] to a subject with erectile dysfunction.

[10] A method for improving erectile dysfunction, comprising administering an effective amount of the microparticles according to any one of [1] to [8] to a non-human animal with erectile dysfunction.

### Effects of Invention

According to the present invention, new microparticles can be provided that enables controlling the expression of a protein and/or a gene related to erectile dysfunction.

### Brief Description of Drawings

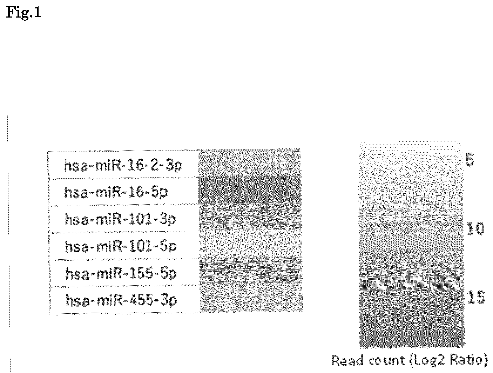

[Fig. 1] A heat map showing the expression levels of miRNAs targeting genes concerning the expression of nitric oxide synthase and expressed in exosomes (microparticles of Example 1) purified from culture supernatant of dental pulp-derived stem cells.

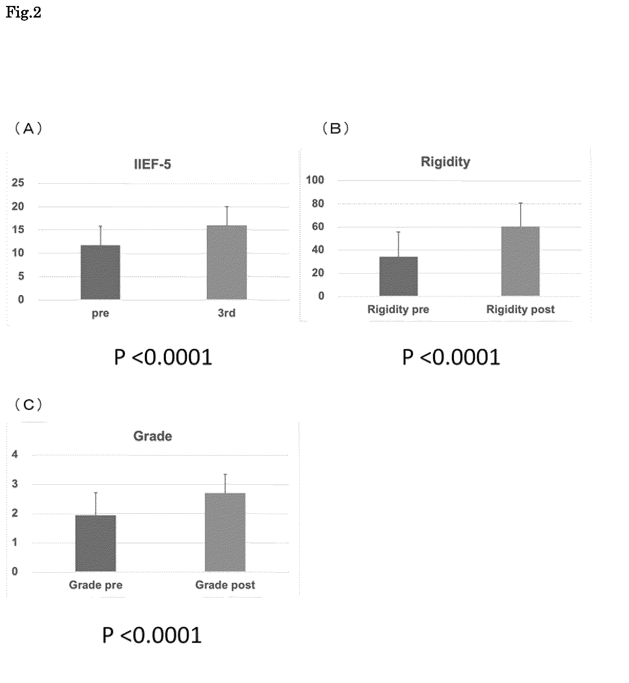

[Fig. 2] Fig. 2 (A) is a graph showing the mean value of the IIEF-5 values of 42 patients before treatment (pre) and the mean value of the IIEF-5 values of the 42 patients after the treatment (3rd). Fig. 2 (B) is a graph showing the mean value of the Rigidity values of the 42 patients before the treatment (pre) and the mean value of the Rigidity values of the 42 patients after the treatment (post). Fig. 2 (C) is a graph showing the mean value of the Grade values of the 42 patients before the treatment (pre) and the mean value of the Grade values of the 42 patients after the treatment (post).

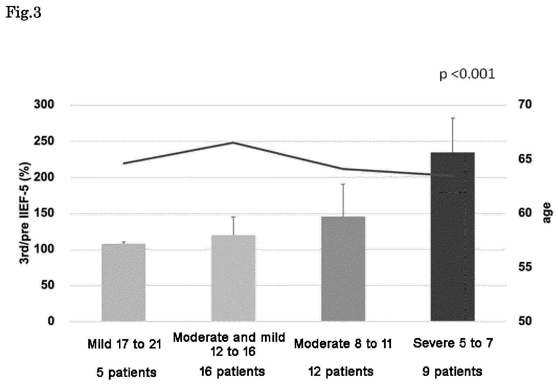

[Fig. 3] A bar graph showing the relationship between the ED severity before the treatment based on the IIEF-5 value and the improvement ratio between the IIEF-5 values; and a polygonal line graph showing the relationship between the ED severity based on the IIEF-5 value before the treatment and the age.

### Description of Embodiments

Hereinafter, the present invention will be described in detail.

Although components described below may be explained based on representative embodiments and specific examples, the present invention is not limited by such embodiments. A numerical range indicated with the term "to" herein means a range including numerical values described before and after the term "to" as the lower limit and the upper limit.

### [Microparticles]

Microparticles of the present invention contain miRNA that controls the production of nitric oxide, and are an agent for promoting the production of nitric oxide.

The microparticles of the present invention enable controlling the expression of a protein and/or a gene related to erectile dysfunction. It is preferable that the microparticles of the present invention consequently enable improving erectile dysfunction. It is more preferable that the microparticles of the present invention enable preventing or treating erectile dysfunction.

Hereinafter, preferable aspects of the microparticles of the present invention will be described.

### <Nitric oxide>

Nitric oxide, which is generated in the body, mainly has a vasodilatation effect. Nitric oxide activates soluble guanylate cyclase in cells to synthesizing cyclic GMP (cGMP), resulting in participating in signal transduction.

The vascular endothelia receive nitric oxide as signals to relax the surrounding smooth muscle, which leads to artery dilation to increase the blood flow. Nitric oxide also acts on the erection of the penis. Nitric oxide is released from the vascular endothelia in the corpora cavernosa of the penis, resulting in the generation of cGMP. The cGMP then relaxes the smooth muscle, leading to dilating blood vessels to promote an erection.

### (Nitric oxide synthase)

Nitric oxide synthase (NOS) synthesizes nitric oxide from arginine and oxygen *in vivo.*

Nitric oxide synthase includes three types, namely neural NOS (nNOS) for producing nitric oxide for signal transduction at low concentration, endothelial NOS (eNOS), and inducible NOS (NOS2 or iNOS) for countering pathogens.

### <Erectile dysfunction>

Erectile dysfunction refers to continuous or recurrent inability to achieve and/or maintain an erection sufficient for satisfactory sexual activity (JSSM Guidelines for Erectile Dysfunction 3rd Edition, edited by the Japanese Society for Sexual Medicine/the Japanese Urological Association).

According to JSSM Guidelines for Erectile Dysfunction 3rd Edition, ED is classified into three types, namely organic ED, psychogenic ED, and mixed ED.

Examples of risk factors of erectile dysfunction in Japanese include twelve factors, namely aging, diabetes, obesity/inadequate exercise, cardiovascular disease/hypertension, smoking, a decrease in testosterone, chronic kidney diseases/lower urinary tract symptoms, neurological disease, operation/injury, mental factors such as depression, drugs, and sleep apnea syndrome. In non-Japanese, dyslipidemia can also be a further risk factor.

### (Mechanism of development of ED)

Risk factors of ED are various, and the mechanisms of development of ED also vary depending thereon.

In a common mechanism of development of ED, the production of nitric oxide decreases locally, resulting in impairing the relaxation of smooth muscle of the corpora cavernosa to cause ED. The mechanisms of development of ED due to the main risk factors are as follows.

In the mechanism of development of erectile dysfunction due to diabetes, the production of nitric oxide locally decreases due to factors such as hypogonadism resulting from vascular disorder, neurological disorder, or diabetes; local infection of the penis; and the onset of Peyronie's disease. The decrease impairs the relaxation of smooth muscle of the corpora cavernosa, resulting in ED.

Examples of known reasons why hypertensive patients develop ED as a complication include disruption of the balance between neurohemodynamic bioactive substances that are essential for maintaining both homeostasis of the blood pressure and erectile function.

Examples of the mechanisms of development of erectile dysfunction due to smoking include vascular endothelial disorder, insufficient blood flow to the penis, and sympathetic nerve stimulation.

Examples of conceivable mechanisms of development of ED due to chronic kidney disease include multiple factors such as insufficient blood flow, neurological disorder, hormone abnormality, renal anemia, the accumulation of endogenous NO synthesis inhibitor (asymmetrical dimethylarginine, ADMA), drugs, and depression.

Examples of suggested mechanisms of development common to both ED and lower urinary tract symptoms include the possibility of overactivity of the sympathetic system, ischemia in the intrapelvic vascular bed, a decrease in NOS/NO, and the upregulation of Rho kinase.

The damage of cavernosal nerves is considered to be the main cause in the mechanism of development of ED due to operation/injury. Even though the nerves themselves are not damaged by an operation, heat during the operation, or local ischemia or inflammation damages the nerves.

### (Therapy for ED)

### (1) PDE5I

Phosphodiesterase 5 inhibitor (PDE5I) is known as an oral drug for ED. PDE5 is an enzyme that decomposes cyclic GMP (cGMP), which is an intracellular second messenger of nitric oxide. PDE5 is abundant in the corpora cavernosa penis. PDE5I inhibits the action of PDE5 competitively to increase the concentration of cGMP in the smooth muscle cells of the corpora cavernosa penis, resulting in relaxation of the smooth muscle of the corpora cavernosa penis, which promotes an erection. For example, sildenafil (Viagra), vardenafil (Levitra), and tadalafil (Cialis) are known.

A therapy for cases of low testosterone is also known using TRT (testosterone replacement therapy) in combination with PDE5I.

### (2) Vacuum erection device

After a vacuum erection device (VED) applies negative pressure to the penis to aspirate blood into the penis, the proximal region of the penis is tied with a rubber band, resulting in blood stasis, which leads to a pseudo erection.

### (3) Injection of prostaglandin E1 into corpora cavernosa

Prostaglandin E1 (PGE1) is injected into the corpora cavernosa penis. According to both the EAU guidelines and the ICSM guidelines in 2015, the injection is recommended as a second-line treatment in the case where PDE5I has no efficacy or in case of contraindication. In cases of poor responsiveness to PGE1, it is assumed that a vascular factor is present. Especially patients with diabetes or metabolic syndrome are poorly responsive. Meanwhile, PGE1 leads to satisfactory erectile response of patients with neurological ED, and exhibits a high efficacy ratio in patients with spinal cord injury or patients after total prostatectomy.

### (4) Low-intensity extracorporeal shock wave therapy

**In** the EAU guidelines, low-intensity extracorporeal shock wave therapy (LI-ESWT) as well as VEDs is adopted as the first-line treatment for cases in which PDE5 inhibitor has no or low efficacy. The therapeutic mechanism thereof is as follows: Low-intensity extracorporeal shock waves expectedly promote neovascularization to improve the vascular endothelial function and the blood flow dynamics. LI-ESWT is applied to vasculogenic ED from this viewpoint.

### (5) Components derived from mesenchymal stem cells

If human umbilical cord blood stem cells are administered to the penis of a man with severe type 2 diabetes, the administration has a useful effect on the erectile function (Exp Clin Transplant. (2010) 8(2): 150-160). The mechanism of the AD treatment is not described in detail in this document.

Meanwhile, the microparticles of the present invention contain miRNA that controls the production of nitric oxide, and are an agent for promoting the production of nitric oxide. Since the microparticles of the present invention enable promoting the production of NO, the microparticles of the present invention relaxes the smooth muscle of the corpora cavernosa penis to promote an erection. The microparticles of the present invention are used for improving erectile dysfunction.

**In** a preferable aspect of the microparticles of the present invention, a NOS-related miRNA group is contained as the miRNAs targeting the genes concerning the expression of nitric oxide synthase, so that the production of nitric oxide synthase is enhanced, resulting in increasing the production of NO.

The microparticles of the present invention may restore a vasodilatation action on the corpora cavernosa by the action mechanism of repair of vascular endothelial cells, which action mechanism is different from the action mechanism of promotion of the nitric oxide synthase expression. The microparticles of the present invention may remarkably promote the production of NO from the vascular endothelia by the synergistic effect of the repair of vascular endothelial cells and the promotion of expression of nitric oxide synthase using the NOS-related miRNA group.

### <Details of microparticles>

The miRNA is contained in the microparticles of the present invention as the active ingredient.

The microparticles of the present invention are derived from the dental pulp-derived stem cells or other cells by secretion, budding, or dispersion from mesenchymal stem cells such as dental pulp-derived stem cells to be exuded, released, or shed into cell culture medium. It is preferable that the microparticles be contained in culture supernatant of dental pulp-derived stem cells. It is more preferable that the microparticles be derived from the culture supernatant of the dental pulp-derived stem cells. The microparticles derived from culture supernatant of dental pulp-derived stem cells does not need to be necessarily acquired from the culture supernatant of the dental pulp-derived stem cells. For example, if microparticles isolated from inside dental pulp-derived stem cells by any method are the same as the microparticles that can be isolated from culture supernatant of dental pulp-derived stem cells, the microparticles isolated from inside dental the pulp-derived stem cells are also the microparticles derived from the culture supernatant of the dental pulp-derived stem cells.

The microparticles derived from the culture supernatant of the dental pulp-derived stem cells or other cells may be used as the culture supernatant containing the microparticles, or the microparticles derived from culture supernatant of dental pulp-derived stem cells may be purified from the culture supernatant for use. It is preferable that the microparticles be purified from the culture supernatant.

The origin of the microparticles can be discriminated by a known method. For example, it can be determined which stem cells among dental pulp-derived stem cells, adipose-derived stem cells, bone marrow-derived stem cells, umbilical cord-derived stem cells, and other stem cells are the origin of the microparticles by the method described in J Stem Cell Res Ther (2018) 8:2. Specifically, the origin of the microparticles can be determined based on the miRNA pattern of the microparticles.

### (miRNA)

In the present invention, the microparticles contain miRNA that controls the production of nitric oxide.

In the present invention, the miRNA (microRNA) is an RNA molecule having 21 to 25 bases (nucleotides). The microRNA enables decomposing target gene mRNA or suppressing mRNA in the decoding step or the transcription step to regulate gene expression.

In the present invention, miRNA may be a single strand (monomer) or a double strand (dimer). In the present invention, it is preferable that miRNA be mature miRNA obtained by cleaving miRNA with ribonuclease such as Dicer.

The sequences of the miRNAs such as has-miR-16-5p described herein are registered in association with the accession numbers thereof in known databases (for example, miRBase database). Those skilled in the art can determine the sequences thereof unambiguously. For example, the accession number of has-miR-16-5p is MI0000070, and the sequence thereof is registered in the miRBase database. Hereinafter, the accession numbers of the miRNAs are omitted.

The miRNA in the present invention however includes variants in which around one to five bases are different from that or those of mature miRNA such as has-miR-16-5p. The miRNAs in the present invention include polynucleotides consisting of nucleotide sequences having identity to the nucleotide sequences of the miRNAs (for example, has-miR-16-5p) or the nucleotide sequences complementary thereto and having the function of the miRNAs in the present invention. The "identity" is the degree of identity between sequences to be compared that are appropriately aligned, and means the incidence (%) of amino acids or nucleic acids of the one sequence that are exactly identical to those of the other sequence. Sequences can be aligned, for example, by any algorithm such as BLAST. For example, the identity is 80%, 85%, 90%, 91%, 92%, 93%, 94%, 95%, 96%, 97%, 98%, or around 99%. For example, the polynucleotides consisting of nucleotide sequences having identity may have point mutations, deletions, and/or additions in the nucleotide sequences of the miRNAs. The number of bases of point mutation is, for example, 1 to 5, 1 to 3, 1 to 2, or 1. For example, the polynucleotides consisting of the complementary nucleotide sequences include polynucleotides that are hybridized with the polynucleotide consisting of the nucleotide sequence of the miRNA under stringent conditions, and has the function of the miRNA in the present invention. Examples of the stringent conditions include, but not particularly limited to, the conditions described in [0028] of Japanese Patent Application Laid-Open No. 2017-184642, and the contents of this publication are incorporated herein by reference.

In the present invention, it is preferable that the microparticles contain the miRNA that controls the production of nitric oxide at higher concentration than the culture supernatant of the dental pulp-derived stem cells. Hereinafter, a preferable aspect of the miRNA that the microparticles contain will be described.

In the present invention, it is preferable that miRNA targeting a gene concerning the expression of nitric oxide synthase be contained as the miRNA that controls the production of nitric oxide. In this case, it is preferable that the microparticles of the present invention be an agent for promoting the expression of nitric oxide synthase.

The miRNA that controls the production of nitric oxide, especially the miRNA targeting a gene concerning the expression of nitric oxide synthase, is effective as a therapeutic drug for the erectile dysfunction. The following miRNAs are known as the miRNAs concerning the production of NO or the expression of NOS.

According to Antioxid. Redox Signal. (2014) 21(18): 2469-2482, the accumulation of Nrf2 in nuclei due to the overexpression of miR-101 increases with HO-1 induction, VEGF expression, and the production of NO derived from endothelial nitric oxide synthase (eNOS).

According to Int. J. Mol. Sci. (2021) 22(12): 6264, miR-155 negatively targets FGF-2 in esophageal adenocarcinomatous cells (EACs) to promote NOS2 in tumor-associated macrophages (TAMs), leading to the control of proliferation, migration, infiltration, and neovascularization. According to Biochem Biophys Res Commun. (2018) 503(2): 452-458, while the introduction of miR-155 strongly induces TNF-α, IL-12, and iNOS, the suppression of miR-155 reduces them. That is, miR-155 increases iNOS.

According to Int. J. Mol. Sci. (2021) 22(12): 6264, miR-16 promotes the activity of induced nitric oxide synthase (NOS2 or iNOS) to increase the production of NO required for maintaining the antitumor microenvironment. miR-16 targets PD-L1 to reduce immunosuppression.

According to Sci. Rep. (2017) 7: 44807, H2S promotes the expression of miR-455-3p to control the stability of eNOS. If miR-455-3p is overexpressed in human umbilical vein endothelial cells (HUVECs), the amount of protein of eNOS increases. On the contrary, the inhibition of miR-455-3p reduces the amount of protein of eNOS.

These miRNAs (miR-101, miR-155, miR-16, and miR-455-3p) concerning the production of NO or the expression of NOS are effective as a therapeutic drug for erectile dysfunction.

It is more preferable that the microparticles of the present invention contain at least one selected from the following NOS-related miRNA group as miRNAs targeting a gene concerning the expression of nitric oxide synthase among these miRNAs (miR-101, miR-155, miR-16, and miR-455-3p) concerning the production of NO and the expression of NOS.
NOS-related miRNA group:
hsa-miR-101-3p, has-miR-101-5p, has-miR-155-5p, hsa-miR-16-2-3p, has-miR-16-5p, and hsa-miR-455-3p.

In this case, it is preferable that the microparticles contain at least one of has-miR-101-5p, has-miR-155-5p, hsa-miR-16-2-3p, has-miR-16-5p, and hsa-miR-455-3p. It is more preferable that the microparticles contain at least one of has-miR-101-5p, has-miR-155-5p, hsa-miR-16-2-3p, and has-miR-16-5p. It is particularly preferable that the microparticles contain at least one of has-miR-101-5p, has-miR-155-5p, and has-miR-16-5p. It is still more preferable that the microparticles contain all of has-miR-101-5p, has-miR-155-5p, and has-miR-16-5p.

It is preferable that the microparticles contain at least one of the miRNAs that target genes concerning the expression of nitric oxide synthase so that the log2 ratio of the read count obtained by analysis using IMOTA be 4.0 or more. It is more preferable that the microparticles contain the at least one so that the log2 ratio of the read count be 6.0 or more. It is particularly preferable that the microparticles contain the at least one so that the log2 ratio of the read count be 8.0 or more. It is still more preferable that the microparticles contain the at least one so that the log2 ratio of the read count be 10.0 or more. It is preferable that the microparticles contain all of hsa-miR-101-3p, has-miR-101-5p, has-miR-155-5p, hsa-miR-16-2-3p, has-miR-16-5p, and hsa-miR-455-3p so that the log2 ratios of the read counts obtained by analysis using IMOTA be 4.0 or more. It is more preferable that the microparticles contain all of has-miR-101-5p, has-miR-155-5p, hsa-miR-16-2-3p, has-miR-16-5p, and hsa-miR-455-3p so that the log2 ratios of the read counts be 6.0 or more. It is particularly preferable that the microparticles contain all of has-miR-101-5p, has-miR-155-5p, hsa-miR-16-2-3p, and has-miR-16-5p so that the log2 ratios of the read counts be 8.0 or more. It is still more preferable that the microparticles contain all of has-miR-101-5p, has-miR-155-5p, and has-miR-16-5p so that the log2 ratios of the read counts be 10.0 or more.

It is further preferable that the microparticles contain both of has-miR-155-5p and has-miR-16-5p so that the log2 ratios of the read counts obtained by analysis using IMOTA be 12.0 or more. It is still further preferable that the microparticles contain has-miR-16-5p so that the log2 ratios of the read counts be 14.0 or more.

It is preferable that the expression level of the NOS-related miRNA group (preferably any one of has-miR-101-5p, has-miR-155-5p, and has-miR-16-5p; more preferably has-miR-16-5p) in the microparticles of the present invention be 1.1 times or more higher than in exosomes obtained from culture supernatant of adipose-derived stem cells or exosomes obtained from culture supernatant of umbilical cord-derived stem cells. It is more preferable that the expression level in the microparticles of the present invention be 1.5 times or more higher than in exosomes obtained from culture supernatant of adipose-derived stem cells or exosomes obtained from culture supernatant of umbilical cord-derived stem cells. It is particularly preferable that the expression level in the microparticles of the present invention be 2 times or more higher than in exosomes obtained from culture supernatant of adipose-derived stem cells or exosomes obtained from culture supernatant of umbilical cord-derived stem cells.

The microparticles of the present invention are an agent for promoting the production of nitric oxide. It is preferable that the amount of nitric oxide produced in any cells (preferably in a vascular endothelial cells of the penis) can be increased to an amount that is 1.2 times or less larger than usual (the amount thereof in untreated cells). It is more preferable that the amount of nitric oxide produced in any cells can be increased to an amount that is 2.0 times or more larger than usual. It is particularly preferable that the amount of nitric oxide produced in any cells can be increased to an amount that is 2.5 times or more larger than usual.

When the microparticles of the present invention are an agent for promoting expression of nitric oxide synthase, it is preferable that the expression level of a nitric oxide synthase gene in any cells (preferably in a vascular endothelial cells of the penis) can be increased to a level that is 1.2 times or less higher than usual (the level thereof in untreated cells). It is more preferable that the expression level of a nitric oxide synthase gene in any cells can be increased to a level that is 2.0 times or more higher than usual. It is particularly preferable that the expression level of a nitric oxide synthase gene in any cells can be increased to a level that is 2.5 times or more higher than usual.

### (Type of miRNAs)

Around 2600 small RNAs are contained in the microparticles derived from culture supernatant of dental pulp-derived stem cells. Around 1800 of these are miRNAs. Among these miRNAs, 180 to 200 miRNAs are contained at high contents. The miRNAs contained in the microparticles derived from the dental pulp-derived stem cells at high contents are characterized by including many miRNAs related to the treatment of cranial nerve disease or erectile dysfunction. This was unknown in the past, and is knowledge that the present inventors have newly found. The miRNAs are very different in this feature from miRNAs contained in microparticles in other mesenchymal stem cells at high contents. For example, few microRNAs related to the treatment of cranial nerve disease or erectile dysfunction are included in miRNAs contained in microparticles of adipose-derived stem cells at high contents, or miRNAs contained in microparticles of umbilical cord-derived stem cells at high contents.

It is preferable that the microparticles contain two or more microRNAs that enable controlling the expression of a protein and/or a gene related to erectile dysfunction (hereinafter also referred to as erectile dysfunction-related microRNAs). It is more preferable that the microparticles contain three or more erectile dysfunction-related microRNAs. It is more preferable that the microparticles contain four or more erectile dysfunction-related microRNAs. It is particularly preferable that the microparticles contain five or more erectile dysfunction-related microRNAs. It is still more preferable that the microparticles contain six or more erectile dysfunction-related microRNAs.

### (Type of microparticles)

It is preferable that the microparticles be one type selected from the group consisting of exosomes, fine vesicles, membrane particles, ectosomes and exovesicles, and microvesicles. It is more preferable that the microparticles be exosomes.

It is preferable that the size of the microparticles be 10 to 1000 nm. It is more preferable that the size of the microparticles be 30 to 500 nm. It is particularly preferable that the size of the microparticles be 50 to 150 nm.

It is desirable that molecules such as CD9, CD63, and CD81 referred to as tetraspanins be present on the surfaces of the microparticles. The molecules may be CD9 alone, CD63 alone, CD81 alone, or any combination of two or three thereof.

Although preferable aspects using exosomes as the microparticles may be described hereinafter, the microparticles used for the present invention are not limited to exosomes.

It is preferable that the exosomes be extracellular vesicles released from cells upon the fusion of multivesicular bodies with cytoplasmic membranes.

It is preferable that the surfaces of the exosomes contain lipid and protein derived from cell membranes of dental pulp-derived stem cells.

It is preferable that the exosomes contain intracellular substances such as nucleic acids (for example, microRNAs, messenger RNAs, and DNAs) and proteins in dental pulp-derived stem cells.

It is known that exosomes are used for communication between cells by transport genetic information from the one cell to the other cell. Exosomes can be easily tracked and targeted in a specific region.

### (Content of microparticles)

The content of the microparticles in the microparticle composition is not particularly limited. It is preferable that the microparticle composition contain 0.5 × 10⁸ microparticles or more. It is more preferable that the microparticle composition contain 1.0 × 10⁸ microparticles or more. It is particularly preferable that the microparticle composition contain 2.0 × 10⁸ microparticles or more. It is more particularly preferable that the microparticle composition contain 2.5 × 10⁸ microparticles or more. It is still more preferable that the microparticle composition contain 1.0 × 10⁹ microparticles or more.

The concentration of the microparticles contained in the microparticle composition is not particularly limited. It is preferable that the microparticle composition contain the microparticles at 1.0 × 10⁸ microparticles/mL or more. It is more preferable that the microparticle composition contain the microparticles at 2.0 × 10⁸ microparticles/mL or more. It is particularly preferable that the microparticle composition contain the microparticles at 4.0 × 10⁸ microparticles/mL or more. It is more particularly preferable that the microparticle composition contain the microparticles at 5.0 × 10⁸ microparticles /mL or more. It is still more preferable that the microparticle composition contain the microparticles at 2.0 × 10⁹ microparticles/mL or more.

Since a preferable aspect of the microparticles of the present invention contains the microparticles in such a large amount or at such high concentration, the aspect enables maintaining a large amount of the miRNA that controls the production of nitric oxide or the miRNA for treating erectile dysfunction.

### <Other components>

As long as the effect of the present invention is not deteriorated, the microparticle composition may contain other components besides the microparticles depending on the kind of an animal to which the microparticle composition is administered and the purpose. Examples of the other components include nutritional components, antibiotics, cytokines, protectants, carriers, vehicles, disintegrators, buffers, emulsifiers, suspending agents, smoothing agents, stabilizers, preservatives, and antiseptics.

Examples of the nutritional components include fatty acids and vitamins.

Examples of the antibiotics include penicillin, streptomycin, and gentamycin.

Examples of the carriers include ingredients known as pharmaceutically acceptable carriers.

The microparticle composition may be the culture supernatant itself or the microparticles themselves of dental pulp-derived stem cells, or may be a pharmaceutical composition further containing a pharmaceutically acceptable carrier or a pharmaceutically acceptable vehicle. The pharmaceutical composition is for use in promoting the administration of the microparticles to an administration subject.

It is preferable that the pharmaceutically acceptable carrier (containing a diluent) do not markedly stimulate an administration subject or deter the biological activity or the characteristics of the compound to be administered. Examples of the carrier include propylene glycol; (physiological) saline solution; emulsions; buffer solutions; medium such as DMEM or RPMI; and cryopreservation medium containing a component for removing free radicals.

The microparticle composition may contain an active ingredient of a conventionally known therapeutic drug for erectile dysfunction. Those skilled in the art can appropriately change the active ingredient depending on the purpose or an administration subject.

Meanwhile, it is preferable that the microparticle composition do not contain predetermined substances.

For example, it is preferable that the microparticle composition do not contain dental pulp-derived stem cells.

It is preferable that the microparticle composition do not contain MCP-1. The microparticle composition may however contain cytokines other than MCP-1. Examples of the other cytokines include cytokines described in [0014] to [0020] of Japanese Patent Application Laid-Open No. 2018-023343.

It is preferable that the microparticle composition do not contain Siglec-9. The microparticle composition may however contain other sialic acid binding immunoglobulin-like lectins than Siglec-9.

It is preferable that the microparticle composition do not substantially contain serum (for example, fetal bovine serum, human serum, or sheep serum). It is preferable that the microparticle composition do not substantially contain conventional serum replacement such as KnockOut serum replacement (KSR).

It is preferable that all the contents (solid content) of the above-mentioned other components in the microparticles be 1% by mass or less. It is more preferable that all the contents be 0.1% by mass or less. It is particularly preferable that all the contents be 0.01% by mass or less.

### <Method for producing microparticles>

The method for producing microparticles is not particularly limited.

Culture supernatant of dental pulp-derived stem cells or other cells may be prepared, followed by purifying microparticles from the culture supernatant to prepare the microparticles of the present invention. Alternatively, culture supernatant of dental pulp-derived stem cells procured by commercial purchase may be purified to prepare the microparticles of the present invention. Furthermore, a composition that has been discarded and contains culture supernatant of dental pulp-derived stem cells may be obtained by transfer (and optionally purified), followed by purifying microparticles therefrom to prepare the microparticles of the present invention.

### (Method for preparing culture supernatant of dental pulp-derived stem cells or other cells)

The culture supernatant of the dental pulp-derived stem cells or other cells is not particularly limited.

It is preferable that the culture supernatant of the dental pulp-derived stem cells or other cells do not substantially serum. For example, it is preferable that the content of serum in the culture supernatant of the dental pulp-derived stem cells or other cells be 1% by mass or less. It is more preferable that the content be 0.1% by mass or less. It is particularly preferable that the content be 0.01% by mass or less.

The dental pulp-derived stem cells may be derived from a human or a non-human animal. Examples of the non-human animal include the same animals (biological species) as the animals to which the microparticles of the present invention are administered. Mammals are preferable.

The dental pulp-derived stem cells used for the culture supernatant are not particularly limited. Stem cells from exfoliated deciduous teeth, deciduous tooth pulp stem cells obtained by other ways, or dental pulp stem cells (DPSCs) are usable. Dental pulp-derived stem cells derived from non-human animals and including pig stem cells from deciduous teeth can be used besides human stem cells from deciduous teeth and human dental pulp stem cells.

Dental pulp-derived stem cells can produce vascular endothelial growth factor (VEGF), hepatocyte growth factor (HGF), insulin-like growth factor (IGF), platelet-derived growth factor (PDGF), transforming growth factor-beta (TGF-β)-1 and -3, TGF-α, KGF, HBEGF, SPARC, other growth factors, cytokines such as chemokines besides exosomes. Dental pulp-derived stem cells can also produce many other bioactive substances.

In the present invention, it is particularly preferable that the dental pulp-derived stem cells for preparing the culture supernatant thereof contain a large amount of miRNA that controls the production of nitric oxide. It is preferable to use stem cells from deciduous teeth. That is, it is preferable that, in the present invention, culture supernatant of stem cells from exfoliated deciduous teeth be used.

As long as the objective treatment can be achieved, the dental pulp-derived stem cells used for the present invention may be natural or genetically modified.

Especially in the present invention, immortalized stem cells of the dental pulp-derived stem cells are usable. The use of immortalized stem cells, which can be substantially proliferated infinitely, enables stabilize the amounts and the composition of biological factors contained in the culture supernatant of the stem cells for a long period of time. The immortalized stem cells of the dental pulp-derived stem cells are not particularly limited. It is preferable that the immortalized stem cells be not cancerous. To the dental pulp-derived stem cells can be added the following low-molecular weight compounds (inhibitors) alone or in combination, followed by culturing the cells to prepare the immortalized stem cells of the dental pulp-derived stem cells.

As long as a TGF-β receptor inhibitor can inhibit the function of transforming growth factor (TGF)-β receptors, the TGF-β receptor inhibitor is not particularly limited. Examples include 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), 2-[(5-chloro-2-fluorophenyl)pteridin-4-yl]pyridin-4-ylamine (SD-208), 3-[(pyridin-2-yl)-4-(4-quinonyl)]-1H-pyrazole, and 2-(3-(6-methylpyridin-2-yl)-1H-pyrazol-4-yl)-1,5-naphthyridine (the above are available from Merck KGaA); and SB431542 (Merck KGaA). A-83-01 is preferably exemplified.

As long as a ROCK inhibitor has the action of inhibiting the function of Rho-binding kinase, the ROCK inhibitor is not particularly limited. Examples of the ROCK inhibitor include GSK269962A (Axon Medchem), Fasudil hydrochloride (Tocris Bioscience), and Y-27632 and H-1152 (the above are available from FUJIFILM Wako Pure Chemical Corporation). Y-27632 is preferably exemplified.

As long as a GSK-3 inhibitor inhibits GSK-3 (glycogen synthase kinase-3), the GSK-3 inhibitor is not particularly limited. Examples include A 1070722, BIO, and BIO-acetoxime (the above is available from Tocris Bioscience).

As long as a MEK inhibitor has the action of inhibiting the function of MEK (MAP kinase-ERK kinase), the MEK inhibitor is not particularly limited. Examples include AZD6244, CI-1040 (PD184352), PD0325901, RDEA119 (BAY86-9766), SL327, and U0126-EtOH (the above are available from Selleck Biotechnology); and PD98059, U0124, and U0125 (the above are available from Cosmo Bio Co., Ltd.).

If the microparticles of the present invention are used for regenerative medicine, an aspect in which the culture supernatant of the dental pulp-derived stem cells or immortalized stem cells thereof, or the microparticle composition containing the microparticles derived therefrom does not contain other somatic stem cells except the dental pulp-derived stem cells or other cells is adopted due to request from the Act on Securing Safety of Regenerative Medicine. Although the microparticle composition may contain mesenchymal stem cells or other somatic stem cells except the dental pulp-derived stem cells or other cells, it is preferable that the microparticle composition do not contain these.

Examples of the other somatic stem cells except mesenchymal stem cells include, but not limited to, stem cells derived from the dermal system, the digestive system, the bone marrow system, and the nervous system. Examples of somatic stem cells in the dermal system include epithelial stem cells and follicular stem cells. Examples of somatic stem cells in the digestive system include stem cells of (all types of cells of) the pancreas and hepatic stem cells. Examples of somatic stem cells in the bone marrow system (except mesenchymal stem cells) include hematopoietic stem cells. Examples of somatic stem cells in the nervous system include neural stem cells and retinal stem cells.

Although the microparticle composition may contain stem cells other than somatic stem cells, it is preferable that the microparticle composition do not contain the stem cells other than somatic stem cells. Examples of the stem cells other than somatic stem cells include embryonic stem cells (ES cells), induced pluripotent stem cells (iPS cells), and embryonal carcinoma cells (EC cells).

The method for preparing the culture supernatant of the dental pulp-derived stem cells or the immortalized stem cells thereof is not particularly limited, and any conventional method is available.

The culture supernatant of the dental pulp-derived stem cells is culture solution obtained by culturing the dental pulp-derived stem cells or other cells. For example, the dental pulp-derived stem cells can be cultured, followed by removing cell components to obtain the culture supernatant available for the present invention. The culture supernatant may be subjected to various treatments (such as centrifugation, concentration, solvent replacement, dialysis, freezing, drying, lyophilization, dilution, desalting, and preservation) for use.

The dental pulp-derived stem cells for obtaining the culture supernatant of the dental pulp-derived stem cells can be sorted by an ordinary method. The dental pulp-derived stem cells can be sorted as adherent cells based on the sizes or the shapes of the cells. The dental pulp-derived stem cells can be sorted as adherent cells or subcultured cells thereof from dental pulp cells collected from exfoliated deciduous teeth or permanent teeth. The sorted stem cells are cultured to obtain culture supernatant, which is available for the culture supernatant of the dental pulp-derived stem cells.

It is preferable that the "culture supernatant of the dental pulp-derived stem cells or other cells" do not contain cultured dental pulp-derived stem cells themselves. It is preferable that, in an aspect, the culture supernatant of the dental pulp-derived stem cells do not contain cells (any types of cells) as a whole. The composition of the aspect is clearly distinguished by this feature from various compositions containing dental pulp-derived stem cells, let alone from dental pulp-derived stem cells themselves. A typical example of this aspect is a composition not containing the dental pulp-derived stem cells and exclusively consisting of the culture supernatant of the dental pulp-derived stem cells.

The culture supernatant of the dental pulp-derived stem cells for the present invention may contain both culture supernatant of dental pulp-derived stem cells from deciduous tooth and culture supernatant of dental pulp-derived stem cells from adults. It is preferable that the culture supernatant of the dental pulp-derived stem cells for the present invention contain culture supernatant of dental pulp-derived stem cells from deciduous teeth as the active ingredient. It is more preferable that the culture supernatant of the dental pulp-derived stem cells for the present invention contain culture supernatant of dental pulp-derived stem cells from deciduous teeth at 50% by mass or more. It is preferable that the culture supernatant of the dental pulp-derived stem cells for the present invention contain culture supernatant of dental pulp-derived stem cells from deciduous teeth at 90% by mass or more. It is particularly preferable that the culture supernatant of the dental pulp-derived stem cells for the present invention be a composition exclusively consisting of culture supernatant of dental pulp-derived stem cells from deciduous teeth.

For example, basal medium or basal medium containing serum and others are available as medium for the dental pulp-derived stem cells for obtaining the culture supernatant. Examples of the available basal medium include Iscove's modified Dulbecco's medium (IMDM) (for example, Thermo Fisher Scientific K.K.), Ham's F12 medium (HamF12) (for example, Merck KGaA or Thermo Fisher Scientific K.K.), and RPMI 1640 medium besides Dulbecco's modified Eagle medium (DMEM). Examples of components that can be added to the medium include sera (for example, fetal bovine serum, human serum, and sheep serum), serum replacements (for example, KnockOut serum replacement (KSR)), bovine serum albumin (BSA), antibiotics, various vitamins, and various minerals.

In order to prepare "the culture supernatant of the dental pulp-derived stem cells" not containing serum, serum-free medium is however preferably used through the whole process, during the last subculture, or during the last several subcultures. For example, the dental pulp-derived stem cells can be cultured in medium not containing serum (serum-free medium) to prepare the culture supernatant of the dental pulp-derived stem cells not containing serum. During one or more subcultures, the last subculture or the last several subcultures using serum-free medium also enable obtaining the culture supernatant of the dental pulp-derived stem cells or other cells not containing serum. Meanwhile, the serum can also be removed from the collected culture supernatant by dialysis or solvent replacement using a column to obtain the culture supernatant of the dental pulp-derived stem cells not containing serum.

Conditions to be commonly used can be applied to culture of the dental pulp-derived stem cells for obtaining the culture supernatant as they are. The culture supernatant of the dental pulp-derived stem cells only have to be prepared in the same way as in the below-described method for culturing cells except the adjustment of the steps of isolating and selecting the stem cells depending on the type of the stem cells. Those skilled in the art can suitably isolate and select the dental pulp-derived stem cells depending on the type thereof.

Special conditions may be applied to the culture of the dental pulp-derived stem cells for producing a large amount of the microparticles such as exosomes. Examples of the special conditions include low temperature conditions, low oxygen conditions, microgravity conditions, and conditions for coculture with some stimulant.

Although, in the present invention, the culture supernatant of the dental pulp-derived stem cells for preparing the microparticles such as exosomes may contain other components besides the culture supernatant of the dental pulp-derived stem cells, it is preferable that the culture supernatant do not substantially contain the other components.

Various additives for preparing the exosomes may however be added to the culture supernatant of the dental pulp-derived stem cells for storage.

### (Preparation of microparticles)

The microparticles can be purified and prepared from the culture supernatant of the dental pulp-derived stem cells or other cells.

It is preferable that the preparation of the microparticles be the separation of fractions containing the microparticles from the culture supernatant of the dental pulp-derived stem cells. It is more preferable that the preparation be the isolation of the microparticles.

The microparticles can be separated from non-associated components based on characteristics of the microparticles, resulting in isolation. For example, the microparticles can be isolated based on the molecular weight, the size, the shape, the composition, or the biological activity.

In the present invention, the culture supernatant of the dental pulp-derived stem cells can be centrifuged to obtain specific fractions containing the microparticles in a large amount (for example, precipitate), which fractions can be collected to purify the microparticles. Unnecessary components (insoluble components) other than the predetermined fractions may be removed. The solvent and the dispersion medium, and the unnecessary components may not be fully removed from the microparticle composition. If the conditions for centrifugation are exemplified, the conditions are 100 to 20000 g and 1 to 30 minutes.

In the present invention, the culture supernatant of the dental pulp-derived stem cells or the centrifuged product thereof can be filtered to purify the microparticles. The filtration enables removing the unnecessary components. The use of a membrane filter having a suitable pore size enables removing the unnecessary components and sterilization simultaneously. The material, the pore size and other properties of the membrane filter used for filtration are not particularly limited. The microparticles can be filtered off on a filter membrane of a molecular weight or size cutoff by a conventionally known method. It is preferable from the viewpoint of collecting exosomes easily that the pore size of the filter membrane be 10 to 1000 nm. It is more preferable that the pore size be 30 to 500 nm. It is particularly preferable that the pore size be 50 to 150 nm.

In the present invention, the culture supernatant of the dental pulp-derived stem cells, the centrifuged product thereof, or the filtered product thereof can be separated with further separation means such as column chromatography. For example, high-performance liquid chromatography (HPLC) using various columns is available. Size-exclusion columns or binding columns are available as the columns.

One or more characteristics or biological activities of the microparticles are usable for tracking the microparticles (or the activity thereof) in fractions in the treatment steps. For example, light scattering, the index of refraction, dynamic light scattering, or an UV-visible light detector are usable for tracking the microparticles. Alternatively, specific enzyme activity is usable for tracking the activity in the fractions.

The method described in [0034] to [0064] of Japanese Translation of PCT International Application Publication No. 2019-524824 may be used as the method for purifying the microparticles. The contents of this publication are incorporated herein by reference.

The final form of the microparticles is not particularly limited. Examples of the form include a form in which a container is filled with the microparticles together with a solvent or a dispersion medium; a form in which a container is filled with gel prepared from the microparticles and gel; and a form of a preparation made from the microparticles solidified by freezing and/or drying; or a form in which a container is filled with the solidified microparticles. Examples of the container include tubes, centrifuge tubes, and bags suitable for cryopreservation. The freezing temperature can be, for example, -20°C to -196°C.

The microparticles of the present invention are advantageous in that the microparticles of the present invention are easily mass-produced as compared with conventional compositions usable as a therapeutic drug or a preventive drug for erectile dysfunction, culture supernatant of stem cells, which culture supernatant was discarded as industrial waste in the past, is utilizable, and cost for discarding culture supernatant of stem cells can be reduced. Especially if the culture supernatant of the dental pulp-derived stem cells is derived from a human, the microparticles of the present invention are also advantageous in that, upon the application of the microparticles of the present invention to a human, the microparticles of the present invention are highly safe from immunological viewpoint, and have few ethical problems. If the culture supernatant of the dental pulp-derived stem cells is collected from a patient with erectile dysfunction, this will enhance the safety of the microparticles of the present invention and reduce ethical problems of the microparticles of the present invention upon the application of the microparticles of the present invention to the patient.

If the microparticles of the present invention are derived from the culture supernatant of the dental pulp-derived stem cells, the microparticles of the present invention are also used for restorative medicine. Especially the composition containing the microparticles of the present invention derived from the culture supernatant of the dental pulp-derived stem cells or other cells are preferably used for restorative medicine. It is known that, in regenerative medicine, premised on stem cell implantation, the stem cells do not play the leading role in regeneration, and humoral components produced in the stem cells restore organs together with the subject's own stem cells. Difficult problems are solved including canceration, standardization, the administration method, the preservation, and the culture method accompanying conventional stem cell plantation, so that restorative medicine can be performed with the culture supernatant of the dental pulp-derived stem cells or the composition containing the microparticles derived therefrom. Since the use of the microparticles of the present invention does not involve cell plantation, neoplastic transformation is less likely to be caused by the use thereof than by stem cell plantation, so that the use thereof is safer than stem cell plantation. The microparticles of the present invention are advantageous in that the microparticles of the present invention can be used having quality standardized uniformly. Since the mass production or effective administration method thereof can be selected, the microparticles of the present invention is available at low cost.

### [Preventive drug or therapeutic drug for erectile dysfunction]

The preventive drug or the therapeutic drug for erectile dysfunction of the present invention contains the microparticles of the present invention.

The term "preventive" as used herein means preventing the onset of disease (corresponding to erectile dysfunction herein) beforehand. The term "therapeutic" as used herein means the alleviation, suppression, or prevention of symptomatic progress in the developed disease and the improvement of the symptoms. It is preferable that the preventive drug or the therapeutic drug for erectile dysfunction of the present invention be a therapeutic drug for erectile dysfunction.

### [Method for improving erectile dysfunction]

A method for improving erectile dysfunction of the present invention includes administering an effective amount of the microparticles of the present invention or an effective amount of the preventive drug or the therapeutic drug for erectile dysfunction of the present invention to a subject with erectile dysfunction.

The step of administering the microparticles of the present invention to a subject with erectile dysfunction is not particularly limited.

Examples of the administration method include the nebulization or the inhalation into the oral cavity, the nasal cavity, or the respiratory tract; an intravenous drip; local administration; and nose drops. It is preferable that the administration method be less invasive. Injection is preferable as the local administration. Electroporation is also preferable for the following reason: Electroporation enables applying a voltage (electrical pulse) to the surface of the skin to bore fine pores in cell membranes to make active ingredients permeate into the dermal layer, which is unapproachable by ordinary care. Examples of the local administration include intracavernosal administration, intravenous administration, intraarterial administration, intraportal administration, intradermal administration, subcutaneous administration, intramuscular administration, and intraperitoneal administration. It is more preferable that the local administration be intracavernosal administration. It is particularly preferable that the administration method in the present invention be intracavernous injection (ICI).

The *in vivo* distribution of the microparticles can be changed by various formulation techniques. Many methods for changing the *in vivo* distribution are known to those skilled in the art. Examples of such methods include the protection of exosomes in vesicles formed of a substance such as protein, lipid (liposomes), carbohydrate, or a synthesized polymer.

The microparticles of the present invention administered to a subject with erectile dysfunction may circulate in the body of the subject to reach a predetermined tissue.

The number of administrations and the administration interval are not particularly limited. The number of administrations can be once or more per month. It is preferable that the number of administrations be 1 to 10 times per month. It is more preferable that the number be 2 to 6 times per month. It is particularly preferable that the number be 4 times per month (around once per week). It is preferable that the administration interval be 1 hour to 2 weeks. It is more preferable that the administration interval be 1 to 10 days. It is particularly preferable that the administration interval be 6 to 8 days (around 1 week). The number of administrations and the administration interval can however be suitably adjusted depending on the biological species of a subject or the symptom of the subject.

It is preferable that the microparticle of the present invention be for use in administration to a subject with erectile dysfunction once or more per week during the therapeutically effective period. If the administration subject is a human, it is preferable that the therapeutically effective period be 1 to 10 weeks, it is more preferable that the therapeutically effective period be 2 to 8 weeks, it is particularly preferable that the therapeutically effective period be 3 to 6 weeks. It is preferable that the therapeutically effective period be set at 3 to 4 weeks and extended to 6 to 8 weeks in the case of a low improvement ratio.

If the culture supernatant of the dental pulp-derived stem cells are used having a concentration of 2.0 × 10⁹ microparticles/ml, with respect to a mouse model, it is preferable that the volume of the culture supernatant be 0.1 to 5 ml per mouse (around 25 g), it is more preferable that the volume be 0.3 to 3 ml, and it is particularly preferable that the volume be 0.5 to 1 ml. If the administration subject is a human, it is preferable that the volume of the culture supernatant be 0.1 to 10 ml per person, it is more preferable that the volume be 0.5 to 5 ml, and it is still more preferable that the volume be 1 to 3 ml.

If the microparticles are used having a concentration of 0.1 × 10⁸ microparticles/µg, with respect to a mouse model, it is preferable that the weight of the microparticles be 1 to 50 µg per mouse (around 25 g), it is more preferable that the weight be 3 to 30 µg, and it is more particularly preferable that the weight be 5 to 25 µg. If the administration subject is a human, it is preferable that the dose of the microparticles be 1 to 100 µg per person, it is more preferable that the dose be 5 to 50 µg, it is still more preferable that the dose be 10 to 30 µg.

A preferable range of the dose to another animal per the body weight of the other animal can be calculated in proportion to the dose to the model mouse per the body weight of a model mouse (around 25 g). The dose can however be suitably adjusted depending on the symptom(s) of an administration subject.

The subject animal (biological species) to which the microparticles of the present invention are administered is not particularly limited. It is preferable that the subject animal to which the microparticles of the present invention are administered be a mammal, a bird (for example, a chicken, a quail, or a duck), or a fish (a salmon, a trout, a tuna, or a bonito). The mammal may be a human or a non-human mammal, and it is particularly preferable that the mammal be a human. It is more preferable that the non-human mammal be a bovine, a pig, a horse, a goat, a sheep, a simian, a dog, a cat, a mouse, a rat, a guinea pig, or a hamster.

The microparticles of the present invention may be used in combination with a conventionally known therapeutic drug for erectile dysfunction. For example, the microparticles of the present invention may be specifically used in combination with PDE5I, which is conventionally known.

### Examples

Hereinafter, features of the present invention will be described further specifically by giving Examples and Comparative Examples or Reference Examples. For example, materials, the used amounts, the ratios, the contents of treatments, and the procedures of the treatments shown in the following Examples can be optionally modified as long as these do not depart from the gist of the present invention. Accordingly, the scope of the present invention should not be interpreted to be limited by specific examples shown hereinafter.

### [Example 1]

### <Preparation of culture supernatant of dental pulp-derived stem cells>

Culture supernatant of human stem cells from deciduous teeth was prepared and collected according to the method described in Example 6 of Japanese Patent No. 6296622 except that DMEM medium was substituted for DMEM/HamF12 medium. Fetal bovine serum (FBS) was added to the cells, which were primarily cultured. The primary culture solution was cultured to obtain subculture solution. The supernatant thereof was collected so that FBS was not contained. The culture supernatant of the human stem cells from the deciduous teeth was prepared. Note that the DMEM is Dulbecco's modified Eagle medium, and the F12 is Ham's F12 medium.

### <Preparation of exosomes>

Exosomes of the dental pulp-derived stem cells were purified from the obtained culture supernatant of the dental pulp-derived stem cells by the following method.

The culture supernatant of the human stem cells from the deciduous teeth (100 mL) was filtered through a filter having a pore size of 0.22 micrometers, and the solution was then centrifuged at 100000 g and 4°C for 60 minutes. The supernatant was decanted. The exosome-concentrated pellet was resuspended in phosphate-buffered saline (PBS). The resuspended sample was centrifuged at 100000 g for 60 minutes. The pellet (around 100 µl) was collected from the bottom of the centrifuge tube as a concentrated sample again. The protein concentration was determined with a Micro BCA Protein Assay Kit (Thermo Fisher Scientific K.K., Rockford, IL). The composition (concentrated solution) containing the exosomes was stored at -80°C.

The composition containing the exosomes purified from the culture supernatant of the dental pulp-derived stem cells was used as the microparticle composition sample of Example 1.

The average particle size and the concentration of the microparticles contained in the microparticle composition of Example 1 were evaluated with the nanoparticle analysis system NanoSight (available from Quantum Design Japan, Inc.).

The microparticles contained in the microparticle composition sample of Example 1 had an average particle size of 50 to 150 nm.

The microparticle composition of Example 1 was an exosome solution at a high concentration of 1.0 × 10⁹ exosomes/ml or more, specifically 2.0 × 10⁹ exosomes/ml.

The components of the obtained microparticle composition of Example 1 were analyzed by a known method, which consequently has shown that microparticle composition of Example 1 does not contain the dental pulp-derived stem cells, MCP-1, or Siglec-9. This therefore has shown that the active ingredients of the microparticle composition of Example 1 are different from MCP-1 and Siglec-9, which were active ingredients of culture supernatant of mesenchymal stem cells, and analogs thereof.

### [Test Example 1]: microRNAs expressed in exosomes

small RNAs contained in the microparticle composition of Example 1 were analyzed by next generation sequencing (NGS). The NGS analysis has identified 1787 microRNAs contained in the microparticle composition (exosomes of the dental pulp-derived stem cells) of Example 1. The obtained results are shown in the following Table 1.

**[Table 1]**

| name | The number of RNAs detected | % |
|---|---|---|
| miRNA | 1787 | 72.35 |
| piRNA | 123 | 4.98 |
| tRNA | 411 | 16.64 |
| other RNA | 149 | 6.03 |
| Total | 2470 | 100 |

### [Test Example 2]: Exploration of microRNAs involved in disease

microRNAs concerning diseases were explored. The microRNAs related to diseases were sampled by IMOTA (interactive multi-omics-tissue atlas). IMOTA is an interactive multi-omics atlas that enables investigating the interactions between miRNAs or mRNAs and proteins and the expression levels thereof in tissues or cells *(*Nucleic Acids Research, Volume 46, Issue D1, 4 January 2018, Pages D770-D775, "IMOTA: an interactive multi-omics tissue atlas for the analysis of human miRNA-target interactions"). It was explored whether the microparticles of the present invention contained microRNAs that control proteins and/or genes related to erectile dysfunction or whether the microparticles of the present invention contained microRNAs that control proteins and/or genes that a therapeutic drug targeted. This Test Example 2 explored miRNAs that targeted the gene involved in the expression of nitric oxide synthase as the protein involved in erectile dysfunction.

miRNAs targeting the genes concerning the expression of nitric oxide synthase and expressed in the exosomes purified from the culture supernatant of the dental pulp-derived stem cells were included in the following NOS-related miRNA group (six miRNAs).
NOS-related miRNA group:
hsa-miR-101-3p, has-miR-101-5p, has-miR-155-5p, hsa-miR-16-2-3p, has-miR-16-5p, and hsa-miR-455-3p.

The expression level of hsa-miR-101-3p was 11.84 as the log2 ratio of the read count obtained by analysis using IMOTA. This was the 54th out of the expression levels of the 1787 microRNAs, which were all of the miRNAs contained in the microparticle composition (exosomes of the dental pulp-derived stem cells) of Example 1 shown in Table 1.

The expression level of hsa-miR-101-5p was 5.13 as the log2 ratio of the read count. This was the 601st out of the expression levels of the miRNAs contained in the microparticle composition of Example 1.

The expression level of hsa-miR-155-5p was 12.17 as the log2 ratio of the read count. This was the 44th out of the expression levels of the miRNAs contained in the microparticle composition of Example 1.

The expression level of hsa-miR-16-2-3p was 8.49 as the log2 ratio of the read count. This was the 162nd out of the expression levels of the miRNAs contained in the microparticle composition of Example 1.

The expression level of hsa-miR-16-5p was 17.69 as the log2 ratio of the read count. This was the first out of the expression levels of the miRNAs contained in the microparticle composition of Example 1.

The expression level of hsa-miR-455-3p was 7.73 as the log2 ratio of the read count. This was the 196th out of the expression levels of the miRNAs contained in the microparticle composition of Example 1.

It has therefore been found that the microparticles of the present invention are usable as an agent for controlling the expression of nitric oxide synthase.

The heat map of Fig. 1 shows the results of comparing the expression levels of the six miRNAs included in the above-mentioned NOS-related miRNA group. The concentration in the heat map are indicated with the log ratios of the lead counts.

Fig. 1 has shown that the microparticles of the present invention contain the miRNAs that controls the production of nitric oxide (miRNAs target the genes concerning the expression of nitric oxide synthase) as the miRNAs concerning improvement in erectile dysfunction at high concentrations. The microparticles of the present invention therefore enable controlling the expression of nitric oxide synthase to regulate the production of nitric oxide, namely the control of the expression the protein and/or the gene related to erectile dysfunction. Consequently, the microparticles of the present invention are useful for improvement, prevention, or treatment of erectile dysfunction.

### [Test Example 3]: Treatment of erectile dysfunction

The culture supernatant prepared in Example 1 and containing the microparticles of the present invention was administered to 42 patients with erectile dysfunction as subjects to be treated, followed by evaluation. These patients were not subjected to ED treatment with other drugs such as PDE5I.

The culture supernatant prepared in Example 1 was directly injected into the penis of each patient. Specifically, a small soft hair band was loosely attached to the root of the penis, followed by injecting 2 ml of the culture supernatant directly into each of the left and right corpora cavernosa through an extra-fine needle once. The culture supernatant was injected into the patient a total of three times at intervals of one week.

The patient was subjected to the therapeutic effect assessments IIEF-5, Rigidity, and Grade before the treatment (pre) and after the start of the treatment to evaluate the therapeutic effects.
(1) International Index of Erectile Function IIEF-5 (Japanese edition): The International Index of Erectile Function IIEF-5 is a type of interview sheet for erectile function. The IIEF-5 value of the patient is derived based on the results of the interview. The IIEF-5 is commonly used for ED screening and the assessment of therapeutic effect on ED.

The severity of ED can be determined based the IIEF-5 value.
Severe ED: 5 to 7
Moderate ED: 8 to 11
Mild to moderate ED: 12 to 16
Mild ED: 17 to 21
Non-ED: 22 to 25

(2) Rigidity: The Rigidity is a type of interview sheet for erectile function. The Rigidity value is derived as the percentage of the erection hardness of a patient as of now to the erection hardness of the patient at the age of 20 (results of the interview).
(3) Grade: The Grade is a type of interview sheet for erectile function that is simpler than the IIEF-5. The Grade value is derived based on the following erection hardness scale (Japanese edition of Erection Hardness Score, EHS): How do you evaluate your erection hardness?
   Grade 1: Penis enlarges but is not hard.
   Grade 2: Penis is hard but not hard enough for insertion.
   Grade 3: Penis is hard enough for insertion but not completely hard.
   Grade 4: Penis is completely hard and rigid.

### <Comparison between assessments of therapeutic effects>

Fig. 2(A) is a graph showing the mean value of the IIEF-5 values of the 42 patients before the treatment (pre) and the mean value of the IIEF-5 values of the 42 patients after the treatment (3rd). Fig. 2 (B) is a graph showing the mean value of the Rigidity values of the 42 patients before the treatment (pre) and the mean value of the Rigidity values of the 42 patients after the treatment (post). Fig. 2 (C) is a graph showing the mean value of the Grade values of the 42 patients before the treatment (pre) and the mean value of the Grade values of the 42 patients after the treatment (post).

Fig. 2 (A), Fig. 2 (B), and Fig. 2 (C) have revealed that the improvement ratios between the values before and after the treatment show results of significant improvements (P < 0.0001) according to all the assessments of the therapeutic effects.

The results have shown a significant positive correlation between the improvement rate between the IIEF-5 values (3rd IIEF-5 value/pre IIEF-5 value) and the improvement rate between the Rigidity values (3rd Rigidity value/pre Rigidity value) (P = 0.003).

The results have shown a significant positive correlation between the improvement rate between the IIEF-5 values (3rd IIEF-5 value/pre IIEF-5 value) and the improvement rate between the Grade values (3rd Grade value/pre Grade value) (P < 0.0001).

The above have shown significant positive correlation between the therapeutic effect assessments.

### <Comparison between therapeutic effects on ED severities>

Fig. 3 is a bar graph showing the relationship between the ED severity before the treatment based on the IIEF-5 value and the improvement ratio between the IIEF-5 values (3rd IIEF-5 value/pre IIEF-5 value); and a polygonal line graph showing the relationship between the ED severity based on the IIEF-5 level before the treatment and the age.

The bar graph of Fig. 3 has shown that as ED before the treatment becomes severer, the improvement ratio between the IIEF-5 values before and after the treatment tends to increase. That is, the treatment has produced a significantly higher therapeutic effect on ED classified as severer based on the IIEF-5 value of ED before the treatment. It has been shown that especially if the microparticles of the present invention are for use in administration to especially a subject having a score of 10 or less based on the International Index of Erectile Function IIEF-5, a significant therapeutic effect is produced.

Note that the polygonal line graph of Fig. 3 has not shown a significant correlation between the ED severity before the treatment and the age.

### <Factors for predicting Responder cases before treatment>

Twenty-two cases in which the improvement ratio between the IIEF-5 values (3rd IIEF-5 value/pre IIEF-5 value) was 135% (median of all the 42 cases) or more were defined as a Responder group. Twenty cases in which the improvement ratio between the IIEF-5 values was less than 135% were defined as a Non-Responder group. The two groups were analyzed. The obtained results are shown in the following Table 2. The 1st means the patients after the first injection of the culture supernatant, the 2nd means the patients after the second injection of the culture supernatant, and the 3rd means the patients after the third injection of the culture supernatant (namely the patients after the completion of the treatment).

The following Table 2 has shown significant differences in age and the IIEF-5 value (pre) between the two groups, namely the Responder group and the Non-Responder group.

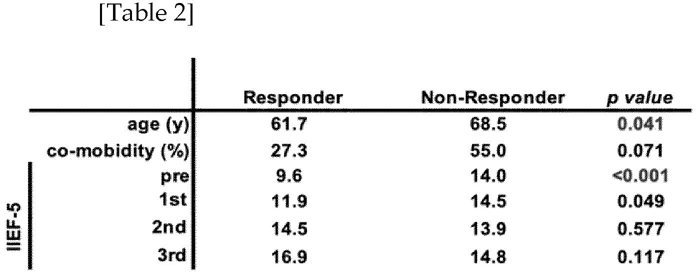

Furthermore, all the 42 types were stratified into the following three groups based on the two factors "age < 70" and "pre IIEF-5 value < 10".

Score 0 ("age ≥ 70" and "pre IIEF-5 value ≥ 10 "): One case of the twelve cases belonged to the Responder group (8.3%).

Score 1 ("age < 70" or "pre IIEF-5 value < 10 "): Twelve cases of the twenty cases belonged to the Responder group (60.0%).

Score 2 ("age < 70" and "pre IIEF-5 value <10"): Nine cases of the ten cases belonged to the Responder group (90.0%).

The above have shown that these two factors are significant factors for prediction before the treatment (p = 0.0001).

The culture supernatant was additionally injected into the remaining one case of Score 2 three times at intervals of one week after the third injection of the culture supernatant, so that the improvement ratio between the IIEF-5 values before the treatment and after the six administrations (6th IIEF-5 value/pre IIEF-5 value) was 222%. That is, the additional administrations finally made all the ten cases of the ten cases in Score 2 belong to the Responder group.

The above have shown that if the microparticles of the present invention are for use in administration to a subject under 70 years of age with severe ED, especially for use in administration to a subject under 70 years of age having a score of 10 or less based on the International Index of Erectile Function IIEF-5, a significant therapeutic effect is produced.

### <Predictive factors in other assessments of therapeutic effects>

First, twenty cases in which the improvement ratio between the Rigidity values (Rigidity post/Rigidity pre) was 155% (median of all the 42 cases) or more were defined as a Responder group. Twenty cases in which the improvement ratio between the Rigidity values (Rigidity post/Rigidity pre) was less than 155% were defined as a Non-Responder group. The two groups were analyzed. The obtained results are shown in the following Table 3.

The following Table 3 has shown a significant difference in the Rigidity value before the start of the treatment (Rigidity pre) between the two groups, namely the Responder group and the Non-Responder group.

**[Table 3]**

| Evaluated by Rigidity | Responder | Non-Responder | *p value* |
|---|---|---|---|
| n | 20 | 20 | |
| age (y) | 63.1 | 66.5 | 0.488 |
| co-morbidity (%) | 30.0 | 50.0 | 0.202 |
| Rigidity pre (%) | 21.8 | 48.0 | <0.001 |
| Rigidity post (%) | 63.3 | 57.8 | 0.682 |

Subsequently, twenty-two cases in which the improvement ratio between the Grade values (Grade post/Grade pre) was 150% (median of all the 42 cases) or more were defined as a Responder group. Nineteen cases in which the improvement ratio between the Grade values (Grade post/ Grade pre) was less than 150% were defined as a Non-Responder group. The two groups were analyzed. The obtained results are shown in the following Table 4.

The following Table 4 has shown a significant difference in the Grade value before the start of the treatment (Grade pre) between the two groups, namely the Responder group and the Non-Responder group.

**[Table 4]**

| Evaluated by Grade | Responder | Non-Responder | *p value* |
|---|---|---|---|
| n | 22 | 19 | |
| age (y) | 63.0 | 67.1 | 0.244 |
| co-morbidity (%) | 31.8 | 52.6 | 0.183 |
| Grade pre | 1.59 | 2.42 | <0.001 |
| Grade post | 2.91 | 2.42 | 0.021 |

The above have shown a significant difference between the Rigidity values of the two groups before the start of the treatment and a significant difference between the Grade values of the two groups before the start of the treatment regardless of age. It has been found that if the microparticles of the present invention are for use in administration to a subject with severe ED, especially a subject having a Rigidity value of less than 40% (preferably less than 30%) or a Grade value of less than 2.0, the microparticles of the present invention produce a significant therapeutic effect.

If the exosomes containing the miRNAs that controls the production of nitric oxide at higher concentration and prepared in Example 1 are substituted for the culture supernatant prepared in Example 1, erectile dysfunction can be further improved.

## Claims

1. Microparticles, comprising:
miRNA that controls production of nitric oxide,
wherein the microparticles are an agent for promoting production of nitric oxide.

2. The microparticles according to claim 1, wherein the microparticles are exosomes.

3. The microparticles according to claim 1, comprising:
miRNA targeting a gene concerning expression of nitric oxide synthase as the miRNA that controls the production of nitric oxide,
wherein the microparticles are an agent for promoting the expression of nitric oxide synthase.

4. The microparticles according to claim 3, comprising:
at least one selected from the following NOS-related miRNA group as the miRNA targeting the gene concerning the expression of the nitric oxide synthase,
wherein the NOS-related miRNA group consists of hsa-miR-101-3p, has-miR-101-5p, has-miR-155-5p, hsa-miR-16-2-3p, has-miR-16-5p, and hsa-miR-455-3p.

5. The microparticles according to claim 1, wherein the microparticles are isolated by purification from culture supernatant of dental pulp-derived stem cells.

6. The microparticles according to claim 5, wherein the microparticles do not comprise components of the culture supernatant of the dental pulp-derived stem cells other than exosomes.

7. The microparticles according to claim 1, comprising the miRNA that controls the production of nitric oxide at higher concentration than the culture supernatant of the dental pulp-derived stem cells.

8. The microparticles according to claim 1 for use in administration to a subject with erectile dysfunction and having a score of 10 or less based on the International Index of Erectile Function IIEF-5.

9. A preventive drug or a therapeutic drug for erectile dysfunction, comprising the microparticles according to any one of claims 1 to 8.

10. A method for improving erectile dysfunction, comprising administering an effective amount of the microparticles according to any one of claims 1 to 8 to a non-human animal with erectile dysfunction.
